# EUROPEAN PATENT SPECIFICATION

(11) **EP 0 871 673 B1**
(45) Date of publication and mention of the grant of the patent: **05.04.2006**
(21) Application number: 96912152.4
(22) Date of filing: 02.05.1996
(51) Int. Cl.: C07K 16/46, A61K 41/00, C07K 16/30, C07K 16/28, C07K 16/40, A61K 39/395

(54) **BISPECIFIC ANTIBODIES IN WHICH THE BINDING CAPABILITY IS REVERSIBLY INHIBITED BY A PHOTOCLEAVABLE MOIETY**
BISPEZIFISCHE ANTIKÖRPER IN DENEN DIE BINDUNGSFÄHIGKEIT DURCH EINE MITTELS LICHT SPALTBARE GRUPPE REVERSIBEL INHIBIERT WIRD
ANTICORPS BISPECIFIQUES DONT LA CAPACITE DE FIXATION EST INHIBEE DE FACON REVERSIBLE PAR UNE GROUPE FISSIBLE PAR EXPOSITION A L'ENERGIE ELECTROMAGNETIQUE

(30) Priority: 03.05.1995 GB 9509004; 04.08.1995 GB 9516606
(43) Date of publication of application: 21.10.1998
(73) Proprietor: BioEnhancements Ltd., Newcastle-Upon-Tyne NE1 4PG (GB)
(72) Inventor: SELF, Colin H., Ponteland, Northumberland NE20 9HJ (GB)
(74) Representative: Kiddle, Simon John
(86) International application number: PCT/GB1996/001066
(87) International publication number: WO 1996/034892

(56) References cited:
- BIOCONJUGATE CHEMISTRY, vol. 3, no. 2, March 1992, WASHINGTON, DC, USA, pages 104-107, XP000262168 V. GOLDMACHER ET AL.: "Photoactivation of toxin conjugates."
- CANCER RESEARCH, vol. 52, no. 19, 15 October 1992, BALTIMORE, MD, USA, pages 5838-5844, XP002010591 L. BAXTER ET AL.: "Pharmacokinetic analysis of the perivascular distribution of bifunctional antibodies and haptens: Comparison with experimental data."
- BIOCHEMICAL AND BIOPHYSICAL RESEARCH COMMUNICATIONS, vol. 201, no. 3, 30 June 1994, DULUTH, MN, USA, pages 1213-1219, XP002010592 S. THOMPSON ET AL.: "Photocleavable nitrobenzyl-protein conjugates." cited in the application
- BIOCHEMICAL SOCIETY TRANSACTIONS, vol. 23, no. 2, May 1995, LONDON, GB, page 156S XP000578198 C. SELF ET AL.: "Studies on photocleavable nitrobenzyl-bovine serum albumin conjugates."
- BIOCHEMICAL SOCIETY TRANSACTIONS, vol. 23, no. 2, May 1995, LONDON, GB, page 155S XP000578199 S. THOMPSON ET AL.: "The modulation of Protein A-IgG(Fc) binding by the reversible addition of 2-nitrobenzyl groups." cited in the application
- PROCEEDINGS OF THE NATIONAL ACADEMY OF SCIENCES OF THE USA, vol. 85, no. 13, July 1988, WASHINGTON, DC, USA, pages 4842-4846, XP002010593 P. SENTER ET AL.: "Anti-tumor effects of antibody-alkaline phosphatase conjugates in combination with etoposide phosphate." cited in the application

## Description

It was been shown that certain proteins can be covalently bound by light labile moieties so that the proteins are prevented from expressing their biological activity until the light labile moieties are cleaved by irradiation (see for example S Thompson et al, Biochemical and Biophysical Research Communications, 201, 1213-1219 (1994) and S Thompson et all, Biochemical Society Transactions, 255S, 23 (1995).

Baxter et al. (Cancer Res., 52, 5838-5844, 1992) describes the use of bispecific antibodies for the treatment of cancer.

The present invention provides an antibody for use in a method of medical treatment, wherein
(a) the antibody is labelled with a member of a binding pair consisting of a ligand and a receptor, so that the antibody is capable of binding to the other member of the binding pair when present on the surface of a target cell which it is desirable to eliminate, and
(b) the antibody is capable of binding to an enzyme which is capable of converting a pro-drug of a cytotoxic drug into the cytotoxic drug, or to a cell of the immune system which can kill said target cell;
wherein the binding of the antibody to the enzyme or cell of the immune system is reversibly inhibited by the presence of a photocleavable moiety.

The invention further provides a bispecific antibody for use in a method of medical treatment, the bispecific antibody comprising a first antibody component which is capable of binding to a member of a binding pair consisting of a ligand and a receptor present on the surface of a target cell which it is desirable to eliminate, and a second antibody component which is capable of binding to an enzyme which is capable of converting a pro-drug of a cytotoxic drug into the cytotoxic drug, or to a cell of the immune system which can kill said target cell;
wherein the binding of the antibody to the enzyme or cell of the immune system is reversibly inhibited by the presence of a photocleavable moiety.

The invention further provides an antibody for use in a method of medical treatment, wherein the antibody is a bispecific antibody capable of binding to
(a) a target cell which it is desirable to eliminate; and
(b) to a virus;
wherein the binding of the antibody to the virus is reversibly inhibited by the presence of a photocleavable moiety.

The invention further provides a kit for use in a method of medical treatment, the kit comprising:
(i) a cell which is a carrier of a cytotoxic agent, a radioactive isotope, a toxin, a virus or boron; and
(ii) an antibody capable of binding to
   (a) a member of a binding pair consisting of a ligand or a receptor present on the surface of a target cell which it is desirable to eliminate; and
   (b) said cell which is a carrier of a cytotoxic agent, a radioactive isotope, a toxin, a virus or boron;
wherein the binding of the antibody to the carrier cell is reversibly inhibited by the presence of a photocleavable moiety.

The antibodies used in this invention may be polyclonally or monoclonally derived.

The term "member of a binding pair" means a member of a pair of entities which will bind to each other when brought into contact. The skilled reader will appreciate that a common and particularly useful binding pairs are antibody and antigen pairs, and ligand and receptor pairs. Many receptors are known to which ligands bind. Thus the antibody of this invention may be labelled with a receptor or, more commonly, labelled with a ligand that will bind to a receptor; for example it may be labelled with hormone that will bind to a hormone receptor. A list of receptors and ligands is given hereinefter.

The antibody may bind to a member of a binding pair because it is an antibody against that member of a binding pair or because it is labelled with the other member of the binding pair. Thus for example an antibody labelled with oestradiol may bind to oestradiol receptors on a cell.

The most suitable members of a binding pair envisaged are cell bound receptors, such as tumour markers.

In a preferred class, the antibody has two active sites. This may be achieved in any convenient manner but it is presently considered most appropriate to join components of two antibodies raised against two different epitopes. The resulting type of antibody is often referred to herein as bispecific antibody. Although normally this type of antibody has two specificies, more than two is also envisaged although less preferred.

Each antibody component may be the whole immunoglobulin (eg IgG) or more suitably may be a part thereof which retains the active site but is free of the Fc region, for example the Fab or Fab'₂.

Most suitably both the first and second antibody components are both parts of antibodies which retain the active sites but are free of the Fc regions, for example the Fab or Fab¹₂.

In certain aspects, this invention makes use of an enzyme capable of producing a cytotoxic agent from a less cytotoxic agent. The enzyme and prodrug are advantageously those known to be suitable for use in ADEPT (antibody directed enzyme pro-drug therapy) (see for example New Antibody Technology and the Emergence of Useful Cancer Therapy, Ed. Richard Begent and Anne Hamblin, pub Royal Society of Medicine Press, especially pages 75-77.

Such enzymes are well blown to the skilled reader and include:
1. Phosphatase (particularly alkaline phosphatase) which can be used to convert less cytotoxic phosphoryrated pro-drugs into the drugs, for example etoposide phosphate, mitomycin phosphate and doxorubicin phosphate may be dephosphorylated to produce etoposide, mitomycin and doxrubicin
2. Carboxypeptidase (particularly G2) which is able to remove glutamic acid residues from pro-drugs in which a glutamic acid residue is used to inactivate the drug
3. β-Glucosidase which can be used to produce cyanide from amygdalin.
4. β-Lactamases (particularly penicillittase and cephalosporinase) can be used to produce viublastine or DAVLBHYD by hydrolysing the β-lactan ring of a pro-drug in which the drug is linked to cephalosporin.
5. Amidase such as a penicillin amidase such as phenoxymethyl penicillin amidase which can produce melphalan or doxorubicin from their acetamide, for example phenoxyacetamide, derivatives
6. Cytosine deaminase which can convert s-fluorocytisine to 5-fluorourscil.
7. Nitroreductase (particularly from E Coli) which converts CB 1954 to an active aklylating agent.

It will be appreciated that by use of this invention enzymes having one or more than one binding site may be employed as desired.

The art is replete with references to target cells and the markers they contain against which antibodies may be raised. Since such cells and markers and the method of raising antibodies are commonly known than form no part of this invention as such. However, it is worthy of mention that carcino embryonic antigen found in human colorectal cancer has had monoclonal antibodies such as YPC2/12.1 raised against them by immunisation with a 108KD glycoprotein by standard procedures. Also antibodies have been raised against the lincocyte common antigen CD45 found in lymphomas and against cytokeratins expressed by carcinoma cells and found for example in draining lymph nodes of patients with carcinoma of the breast.

Other cell tumour markers include (i) viruses CBV and HPV, (ii) mutated or altered genes ras, p53, connexin 37. frame shift mutations (FAP); (iii) normally silent genes: MAGE (melanoma); iv unpredulated: PEM (MUCI product); different antigens: tyrosinases. Melan. Mart 1, gp 100, c-erb B2. CEA, HER2, EGFR, CA125, antibody from B cell lymphomas, T-cell receptors.

The following are also of note: (i) with respect to anti CEA for, for example, Colorectal cancers see J Natl Cancer Inst 81, 688-696 (1989) and Cancer lmmun Immunother 25, 10-15 (1987), Cancer Res 45, 5769-80 (1985), Cancer 63, 1343-52 (1989); (ii) AntiC14(LeY), Ten Feizi Biosci Rep 3, 163-170 (1993); (iii) Anti 791T/36 (TAG 72gp), ----J Cancer 43, 6153-60 (1989),Cancer Res 46, 5524-5528 (198G), Cancer Res 49, 6153-60 (1989) (Xoma): (iv) Anti CEA (CEM 231) J Immunol 141, 1053, 4060 (1988); (v) Anti-TAG 72 (CC49), Cancer Res 48, 4583-96 (1988), 50, 1291-98 (1990), 51,2965-72, 46, 2325-38 (1986); (iv) Anti KS1/4, Cancer Res 50, 3540-44 (1990), 44,681-87, Cancer Immun lmmunther 28, 171-8; (vii)MAbB3, Cancer Res 51, 3781-37B7; (viii) C242. J. Clin. Inves. 90,405-11 (1992); (iv) P-Glycoprotein, PNAS 83, 7785-89 (1986), Cancer Res 48, 1926-9 (1988); and (x) the antibodies referred to against, inter alia, colorectal, gastric and ovarian cancers, referred to in J. National Cancer Institute 81. 688-696 (1989).

The term "photocleavable moiety" means any agent attached to the antibody which can be removed on exposure to electromagnetic energy such as light energy of any desired variety whether visible, UV, X-ray or the like (e.g. microwave).

Suitable photocleavable moieties are well known from the art, for example Biological Applications of Photochemical Switches. Ed H Morrison, Biiorganic Photochemistry Series. Vlumne 2, J, Wiley & Sons, especially Chapter 1, Section 4, pages 34-50.

The photocleavable moiety employed is largely a matter of choice but it has been found to be particularly easy to employ a reagent which couples to hydroxy or amino residues present in the antibody. Thus phosgene, diphosgene, DCCI or the like may be used to generate photocleavable esters, amides, carbonates and the like from a wide range of alcohols. Generally substituted by arylalkanols are employed, particularly mitorphenyl methyl alcohol, 1-nitrophenylethan-1-ol and substituted analogues. The methods of the publications of S Thompson et al referred to hereinbefore may be employed. Generally the cloaking of the antibody will take place in aqueous solution at ambient temperature. Cosolvents such as dioxane may be employed if desired. The chemistry will be familiar to the skilled worker who derivarises macromolecules such as antibodies. The references referred to in the preceding incorporated publication may be consulted if desired.

In the treatment of tumours using a pro-drug, an antibody capable of binding to the tumour cell and to a suitable enzyme is administered to the subject and irradiated, the enzyme (if non endogenous enzyme is to be employed) is administered to the subject and the pro-drug is administered (which is converted to the more cytotoxic drug in vivo).

The antibody is not normally irradiated until after administration and distribution within the body, for example 2 hrs to 10 days after irradiation, for example between days 1 to 8, example on day 3 or 4.

Administration of the enzyme will generally take place at about the time irradiation, either shortly before, cuncurrently or shortly after, for example within 2 hours ofirradiation. The pro-drug is not normally administered until after irradiation, for example 2hrs to 7 days later, for example on day 1 or 4. The time delays following administration of the antibody and enzyme allow for clearance of undesired sequestered material from other parts of the body. The delays may be omitted but this is not presently considered to be preferred.

The areas to be irradiated will be where the tumour is located or where a site is at risk (such as an adjacent lymph node). Most suitably the tumour to be treated will be one readily illuminated because of its location, for example one adjacent to an area which can be reached by an appropriate source of light energy directly or via a light guide such an optical fibre. Thus rumours occurring in the lung, gastro-intestinal tract, urinary tract, testes, ovaries and body cavities, are particularly appropriate sites. Tumours in organs such as the lung, stomach, bowel and prostate are readily irradiatable without needing to penetrate the body whereas other areas such as the liver and kidneys are accessible by minimally invasive surgical methods if visible or UV tight (such as UV-A) is employed. Use of X-rays also allows opaque tissues to be treated.

Since the less toxic pro-drug is converted to active cytotoxic agent only in the locality of the tumour to be treated this invention enables the cancerous tissue to be treated with less toxic effects in remote tissues than would occur if an effective amount of cytotoxic drug were given.

In this invention the photocleavable moiety may be located at or about the active site of the antibody. This may be achieved by incorporation of a single moiety at an active site for example by the methods of P Mendel et al., J. Am. Chem. Soc., 113, 275-2760 (1991).

In a preferred manner the photocleavable moiety may be incorporated into the antibody by coating the antibody with one or more usually more than one such moieties. Suitable methods include those of the publications by S Thompson et al referred to hereinbefore. Thus preferred antibodies (including fragments) of this invention are those which contain more than one photocleavable moiety, for example 2-25, more aptly 3-15. for example 4-8. If only one such moiety is included it is generally at the active site.

Bispecific antibodies as used in this invention may be prepared from individual antibodies, or preferably from the Fab or Fab¹₂ fragments of the individual antibodies by standard methods

The antibodies as used in this invention which also comprise a ligand may be prepared by labelling the antibody with the ligand either before or after the photocleavable moiety is bound to the antibody.

In the instances where only the relevant binding site can be effected by coating, then the coating procedure may be performed on the antibody. (This may be where other reactive sites are absent or where they are protected, for example by absorption on an immunoabsorbant column). In the more common case where coating could effect, for example the other antibody component of the bi-specific antibody, then the coating is best performed on one component before coupling to form the bi-specific antibody.

The bispecific antibody may then by prepared by linking the first antibody component and the reversibly inhibited second antibody component in a manner known in the art for preparing befunctional antibody. For example see the disclosures on bifunctional antibody preparation in "Monoclonal Antibodies. Production, Engineering in Clinical Applications, Postgraduate Medical Science, Edited by Mary, A. Ritter and Heather M. Ladyman, Cambridge University Press (1995) ISBN 0521473543; in particular the section by S Songsivilai and P J Lachmann at pages 121-135.

The location of specific biological cells is critical to both life systems and industrial processes. For example, bodily defence systems work by the localisation of specific cells such as T-cells, polymorphonuclear cells, macrophages, etc. to sites where foreign agents or aberrant cells are identified. A great deal of interest has been shown in developing means of increasing the attraction of cells to such sites, especially when the aberrant cells are tumour cells or infected cells such as cells infected with the immunodeficiency virus (Berg et al, Proceedings of the National Academy of Sciences of the United States of America, 88(11):4723-7,1991). Ligands, such as steroids and other substances which bind cellular membranes, receptors, enzymes, adhesion molecules, nucleic acids and antibodies are all classes of substances which can be used for such cell localisation.

Bispecific reagents, which have an affinity for both the site and the cell to be localised at the site may be made from these specific combining partners. Indeed, the specific combining abilities of specific antibodies against tumour cells on one side and the cells intended for localisation on the other side have already been used in this regard. Bispecific (and trispecific - Tutt et al, Journal of Immunology, 147(1):60-69, 1991) antibodies have been made for such localisation. These are composed of an antibody binding site against an antigen epitope of the tumour cell which, in the bispecific case, is connected to other antibody binding site against an antigenic epitope on the cell type (or virus - Mady et al, Journal of Immunology, 147(9):3139-3144, 1991) that is it is intended to localise at the tumour site. The specificity of the antibodies thus used is helpful in achieving localisation, however, it is important to minimise localisation of cells such as cytotoxic and inflammatory cells at sites other than those specifically targeted. A means of achieving greater localisation than obtainable by antibody alone would confer a distinct advantage on the approach. This has now been achieved as disclosed in this specification. This is done as follows.

A bispecific reagent is made having a binding site for the site to which localisation is to be directed and a second binding site for cells which are to be localised at the site, however, at least one of these binding sites is inhibited from being able to bind until activated. Such inhibition may be achieved by means of coating the second binding site with a photolytically cleavable residue which inhibits the site from cell binding until photolytically cleaved. Thus reagents of the following forms may be employed:
(i) the reagent has its specific binding affinity for the site to which localisation is to be directed uninhibited whilst having its binding affinity for the cells to be localised reversibly inhibited;
(ii) the reagent has its specific binding affinity for the site to which localisation is to be directed and also its specific binding affinity for the cells to be localised reversibly inhibited.

An example of the use of such a localising reagent is as follows:-
A bispecific antibody is made comprising an antibody site against a tumour cdl and a reversibly inhibited antibody site against a cytotoxic cell such as a natural killer cell may and is given to an individual with a tumour site against which the bispecific antibody will bind. It is allowed to localise at the site. In common with other bispecific antibodies other fractions will be taken up non-specifically in the reticuloendothelial system and specifically elsewhere such that it exists in spleen, liver, kidneys etc. The site to be treated is then exposed to light (such as UV-A light) causing activation of the anti-cell antibody binding site of bispecific antibodies bound at that location These cells are then localised at that site and have their effect.

There are number of advantages of the approach. It is well known that antibody directed therapy is not as specific as desired (Bagshaw K D, page 79 of New Antibody Technology and the Emergence of Useful Cancer Therapy, Ed. Begent & Hamblin, published by the Royal Society or Medicine Press Ltd. 1995). The approach of this specification builds upon this specificity, At sites at which the bispecific antibody is not activated, the cells will not be specifically localised by the antibody, however, within the sites illuminated the antibodies will be activated. Importantly, not the whole site illuminated will necessarily thus contain an equal covering of activated antibody but it will be differentially present across the site in relation to the density of antigenic epitope found there. A limited degree of non-specific binding will be expected and antibody doing this within the illuminated area will be activated. However, the non-specific binding will be a small fraction of that had an uninhibited bispecific antibody been employed. Another advantage of the method is that with a conventional bispecific antibody, as soon as it enters the individual (by for example injection into a vein) it is able to bind to the cells to be localised. This was highlighted as a problem in the use of antibodies containing anti-CD3 specificity by Barr et at (Int. J. Cancer, 43, 501-597; 1991) stating that "Specific antibodies containing anti-CD3 specificity will not be suitable for systemic administration in humans since they will be absorbed by all peripheral lymphocytes". This would clearly decrease the ability of the antibody to penetrate to the sites to be treated.

The localising cells of this specification includes viruses. As far as the localising cells are concerned they may be (i) cells already present within the individual or either normally or caused to increase before treatment by, for example, causing inflammation to occur at an area of the body or (ii) cells added from another immune or non-immune individual or previously taken from the individual himself and optionally treated (by culture to enhance their effectiveness, for example: lymphokine activated killer (LAK) cells (Nippon et al, Journal of the Nippon Medical School, 58 (6): 663-672); cloned cells such as T-cells may also be used. Additionally, cells may be employed as carriers of cytotoxic agents or substances, for example the encapsulation of recombinant human IFN gamma in human red blood cells targeted against adenocarcinoma (Chokri et al, Research in Immunology, 143 (1): 95-99, 1992). Similarly a radio-active isotope, a toxin such as diphtheria toxic, boron (for subsequent boron capture treatment) may be carried or the cell itself may be virally infected.

The antibodies specific for the foreign agents and cells to be localised may be obtained by standard means. In many instances it is preferable that they are of low inherent immunogenicity themselves. Humanised antibodies can, therefore, be used with good effect in bispecific antibody technology (Shalaby et al, Jorunal of Experimental Medicine, 175: 217-225, 1992). It is preferable that they be monoclonal antibodies but polyclonal antibodies may also be employed Tumour cell targets include those described in the earlier patent application (ours - incorporated) some of which have already been used in targeting of bispecific antibodies (ovarian carcinoma - Segal et al, Immunobiology, 185; 390-402, 1992); breast cancer (Shi et al, Journal of Immunological Methods, 141, (2): 165-75); colon carcinoma (anti-CEA) (Jantscheff et al, Journal of Immunological Methods, 163 (1): 91-97, 1993); colon carcinoma (Beun et al, Journal of Immunological Methods 150 (6): 2305-2315, 1993); leukemic B-cells (Bohlen et al, Journal of Immunological Methods, 173 (1): 55-62, 1994); small cell lung cancer (Azuma & Niitani, Japanese Journal of Thoracic Disease, 29 (9): 1132-1137, 1991); adenocarcinoma (Chokri et al, Research in Immunology, 143 (1): 95-99, 1992); ovarian and breast carcinomas (Journal of Experimental Medicine, 175: 217-225, 1992).

Similarly, many different antibodies have been made against suitable cells to be localised, for example: T-cells (review) (Bolhuis et al, Journal of Cellular Biochemistry, 47: 306-310, 1991); cytotoxic T-cell clones (Haagen et al, Clinical and Experimental Immunology, 90 (3): 368-75, 1992); cytotoxic T-cells and activated peripheral blood lymphocytes (van Ravenswaay et al, Gynecological Oncology, 52 (2): 199-206, 1994); CD3+ lymphocytes (Malygin et al, Immunology, 81 (1), 92-95, 1994); CD 16+ lymphocytes (Nitta et al, Immunology Letters, 28 (1): 31-37, 1991); Fc gamma R111, the low affinity Fc gamma receptor for polymorphonuclear leucocytes, macrophages and large granular lymphocytes (Garcia de Palazzo et al, International Journal of Biological Markers, 8 (4): 233-239, 1993), B-lymphocyte markers (Demanet et al, International Journal of Cancer - Supplement, 7 67-68, 1992); myeloid cells (Ball, et al, Journal of Hematotherapy, I (1): 85-94, 1992); T Lymphocyte CD2, CD3, CD4, CD8 (Tutt et al, Journal of Immunology, 147 (I) 60-69, 1991); dengue virus (Mady et al, Journal of Immunology, 147 (9) 3139-3144, 1991), lymphokine activated killer (LAK) cells (Nippon et al, Journal of the Nippon Medical School, 58 (6): 663-672); NK cells or monocytes (Curnow et al, Scandinavian Journal of Immunology, 36 (2): 221-231. 1992).

So-called bispecific antibody "forks" have been described by (Ring et al, Cancer Immunol. Immunotherapy, 39 41-48; 1994). These are antibodies which have two specificies against two different markers on a tumour cell. For the fork to bind a tumour cell will require greater specificity than a single site would require and also the avidity of the antibody for the cell would be higher than with a single binding.

Employing the coating technology of the present specification it would be possible to coat one or both binding sites which might interfere with the localisation of the fork and only uncover it in the location of the tumour cell. That would reduce the chance of the antibody being removed by binding cells binding a single specificity on the way to the tumour cells.

Bispecific antibodies may be made in a number of ways, for example:

By chemically coupling an antibody of one specificity to another antibody of the other specificity by any of the wide variety of chemical means available for such coupling (such as glutaraldehyde, or preferably, one of the many more specific cross linkers such as SPDP - (N-Succininimidyl-3-[2-pyridyldithio] propionate). In this specification antibody means an intact antibody containing the usual number of binding sites and an Fc region or any fragment thereof which contains the antigen binding site (such as Fv, Fab, (Fab'). Suitable methods for the production of bispecific antibodies are described by H. Paulus, Behring Inst. Mitt., 78, 118-132, 1985. Small antibody fragments have particular advantages and may be prepared as described by Holliger et al, Proceedings of the National Academy of Sciences of the United States of America. 90 (14): 6444-6448; 1993).

In addition bispecific antibodies can be produced by bringing together two or more antibodies by means of cross-linking binders such as anti-antibodies, lectins or reagents such as protein A (Ghetie & Mota, Mol. Immunol. 17, 395-401; 1980)

The heavy and light chains of the antibodies can be separated and allowed to recombine with each other, some chains thereby combining with chains of the other antibody forming bispecific antibodies (Paulus, Behring Inst. Mitt., 78, 118-132, 1985; Lebegue et al, C.R. Acad. Sci. Paris, Serie 111, 310, 377-382, 1990);

By the formation of quadromas from the two hybridomas synthesising the two antibodies from which a bispecific antibody is required (Suresh et al, Methods in Enzymology, 121, 211, 1986; Bos R, Nieuwenhuitzen W, Hybridoma, 11 (1): 41-51, 1992);

By genetic recombinant means such as described in COS-1 cells for the production of a bifunctional murine:human chimeric antibody (De Sutter & Fiers, Molecular Immunology, 31 (4): 261-267).

The bispecific reagent may have any specific binding partner as either one of its specificities. The features required by a specific binding partner to be useful in the manner of this invention are as follows: that it can be coupled to another binding partner and still retain significant specific binding affinity for the other member of its binding pair; that it or a binding partner to which it is intended to be coupled may be reversibly inhibited from binding to the member of its own binding pair.

Classes of such specific binding partners are within, for example: antibodies, enzymes, ligands and receptors including biotin and avidin, adhesion molecules (such as ICAM-1, E-Selectin, VCAM-1, 1-SELECTIN and Endothelin-1), lectins, nucleic acids (such as DNA & RNA).

The coated reagent may have any number more than one of its sites reversibly inhibited. If one or more of the sites are to remain uninhibited this may be achieved by treating the sites to be inhibited before subsequently coupling them together with uninhibited sites or by taking advantage of differences in susceptibility to inhibition by different sites either as a result of their natural formation or by specifically reducing the likelihood that a site will be inhibited by adding the inhibitory substance in the presence of a specific combining partner for the site. For example, in the case where a bispecific reagent is required between and antibody of specificity K for a tumour cell marker and specificity L for a T-cell and in which the L specificity was required to be inhibited whilst the K was not, a bispecific antibody could be made by any of the art methods and that bispecific antibody bound with antigen K whilst the sites against L were inhibited. The reagent used for causing inhibition of the sites could then be withdrawn and the bispecific antibody separated from antigen K to provide the species required.

The antibodies described herein are capable of binding to a first cell which it is desirable to eliminate for example a tumour cell, cell of a parasite such as the malaria parasite or the like. Most desirably the first cell is a tumour cell, for example as hereinbefore named.

The antibodies described herein may also be capable of binding to a cell capable of killing the first cell when localised in the environs of the first cell. Although the term cell is to interpreted broadly also to include viruses, aptly the cell is a prokaryotic or eukaryodc cell and is most favourably a mammalian cell and is preferably a human cell. Most preferred are cells of the human immune system which can kill undesired cells, for example those killer cells named hereinbefore.

The antibodies bind to the first cell via a cell bound or receptor ligand, such as tumour markers so that the antibody binds to tumour cell possessing said marker.

### Example A

### The use of a reversibly inhibited bispecific antibody to specify the location of killing of tumour cells

The monoclonal antibody anti-CD-3 OKT3 (from the American type culture collection) against T-cells is taken and treated with 1-(2-nitrophenyl)ethanol (NPE) as follows:

First, 0.66g 2 nitroaceloacetophenone is dissolved in a round bottomed flask in 7ml of industrial methanol in the presence of NaBH4. The contents are swirled continuously to mix for 60 minutes. Ten ml H2O are then added to the flask with one drop 1M HCl. Ten ml of ethyl acetate is added and the mixture shaken thoroughly in a separating funnel. The bottom aqueous layer is run off and discarded. The washings are repeated with another 10ml H2O. The top layer is collected and placed in an evaporating flask with MgSO4 to remove all traces of water. The preparation is passed through filter paper and the clear NPE cluare collected. The methanol is then removed on a rotary evaporator at 60C leaving a small quantity of viscous material. This is left overnight open in a fume cupboard. 31.3 µl of di-phosgene is added to a solution of 44mg NPE and 20.6µl of pyridine in 1ml of dry dioxan. A white precipitate immediately forms and the reaction is allowed to go to completion over 15 minutes. The reaction mixture is then evaporated in a stream of nitrogen for 45 minutes to remove unreacted material and the off-while nitrobenzyloxycarbonyl chloride (NPE-COCI) is resuspended in 1ml of dioxan, The following steps in this example are then carried out with protection of the light-sensitive reagents to right. One hundred µl of this was then added to 15ml of the anti-CD-3 OKT3 antibody dialysed at a concentration of 0.5mg/ml against 0.1M NaHCO3 al pH8.3 and gently rotated for 4hr. The solution was then dialysed against 0.9% NaCl. Antibody retaining binding to T-cells is then removed by exposing the treated antibody to a suspension of T-cells at 4C, incubating, removing the supernatant solution and then purifying it by HPLC chromatography. The antibody is then conjugated with a monoclonal antibody against human colon carcinoma cells LoVo by means of standard treatment with a heterobifunctional cross linker such as (N-succinimidyl-3-[2-pyridydithio]proprionate) (SPDP) and further purified to obtain a purified preparation of 1:1 conjugates by HPLC.

Following the approach by Barr (Int. J. Cancer, 43, 501-507; 1991) the cytotoxicity of the bispecific antibody against 5 1Cr-labelled LoVo cells is then assessed in microtitre plates by means of the standard cytotoxicity assay of Brunner et al (In: B. Bloom & J.R. David (eds), In vitro methods in cell-mediated and tumour-immunity, p.423, Academic Press, New York (1976). Those wells which receive cytotoxic T-cells, labelled LoVo cells, NPE-inhibited bispecific antibody and are irradiated with UV-A light by exposure to a Spectroline EN-16/F UV lamp (Spectronics Corporation, Westbury, New York) for 15 minutes show greater cytotoxicity than the same mixture without irradiation or the mixture without the NPE-inhibited antibody but with 15 minute irradiation. Enhanced cytotoxicity is also observed over the controls if the bispecific antibody is first exposed to the UV-A source for 15 minutes while held in quartz-glass cuvettes before addition to the cytotoxicity test wells.

### Example B

### The use of a reversibly inhibited bispecific antibody in the rapid determination of low levels of micro-organisms in a water sample

Two monoclonal antibodies (P & Q) are obtained by standard means against the micro-organism E.coli which are shown to be able to both bind the micro-organism simultaneously. This is done by standard means which involve briefly: (1) coating a microtitre plate with monoclonal antibody P (2) adding a suspension of the micro-organism, incubating this and then washing off unbound material and (3) adding the monoclonal antibody Q [which had been suitably labelled with alkaline phosphatase by means of the Pierce & Warriner (UK) Maleimide Alkaline Phosphatase Conjugation Kit (1995 cat number 31492)] allowing it to bind, washing off unbound material and (4) determining the alkaline phosphatase remaining. A substantial enzyme reactivity compared to other immunoassays employing this plate format, proves that the two antibodies can bind the micro-organism simultaneously.

The following part of this example are then carried out with protection of the light-sensitive reagents from light. A Monoclonal antibody R is then obtained against bovine serum albumin (BSA) and then conjugated with NPE as in Example I of this specification. Antibody still able to react with BSA is removed by means of exposure to an immunoabsorbent, BSA-beaded agarose (Sigma Chemical Co. Ltd., 1995 cat. no. A3790). The remaining conjugate is then coupled to monoclonal antibody P employing SPDP and purifying the 1:1 conjugates by means of HPLC, again as in Example 1 of this specification. This conjugate Z is then used in the test as follows:

Polypropylene cuvettes of thickness 1cm, 2cm width and 5cm high are taken and 1ml of a 1% solution of BSA added into their bottoms in a coating buffer of 50mM bicarbonate pH 9.3 and left for five minutes for coating to take place. The solutions are then withdrawn by means of a pastuer pipette and the cuvettes washed six times with a washing solution of 50mM tris pH 7.4 plus 0.02% Tween 20). 0.2ml of a 20µg/ml solution of conjugate Z are then placed in the cuvettes followed by eight ml of the water samples to be tested and standards containing known numbers of E.coli organisms, with mixing. The mixtures are then incubated for five minutes at room temperature to allow binding between E.coli present and conjugate Z. After this time the broad face of the cuvettes are exposed to UV-A light of the same intensity as above for a further fifteen minutes with gentle agitation. Further light protection is then unnecessary. The solutions are removed and the cuvettes washed six times with washing solution. 2ml of a 10mM solution of substrate para-nitrophenol phosphate in 50mM bicarbonate buffer pH 10.3 containing 3.3mM MgC12 are then added and the cuvettes incubated until a suitable change at their absorption at 405 nm had taken place for the cuvette with the highest standard. A graph is plotted of absorption change against E.coli amount for the standards and the unknown samples read off against this. The results are superior than those obtained from a parallel example in which uninhibited bispecific antibody is added for in this case some bispecific antibody combines with the BSA in the cuvette before the E coli in contrast to the case with the inhibited bispecific antibody where it remains in solution for a sufficient time for the E.coli binding to take place in the favourable solution environment.

### EXAMPLE 1

### Inhibited Antibody Against Alkaline Phosphatase and CEA

a) A polyclonal antibody against alkaline phosphatase (Sigma Chem. Co. Ltd., 1994 catalogue no. p5521) by standard procedures and finally purified by means of an alkaline phosphatase affinity chromatography column.
b) 1-(2-nitrophenyl)ethanol was treated with di-phosgene in dioxane to yield nitrophenethyloxycarbonyl chloride (S Thompson et al, Biochem. Biphys. Res. Comm., 201, 1213-1219 (1994).
c) A monoclonal antibody against tumour antigen CEA was obtained in standard manner from a hybridoma cell line.
d) Aliquots of nitrophenylethyloxycarbonyl chloride were added to 1mg of purified anti-alkaline phosphatase in 1 ml of 0.1 M NAHCO₃ solution. The resulting material was dialysed against 0.9% sodium chloride solution.
e) The monoclonal antibody against tumour antigen CEA was conjugated to the reversibly inhibited anti-akaline phosphatase antibody by means of the heterobifunctional cross-linker 3-(2-pyridyldithio)propionic acid N-Hydroxysuccinimide ester (SPDP - Sigma Chemical Co. Ltd 1994 cat no. P3415) as follows: the anti-alkaline phosphatase antibody (2mg, 12 mg/ml) and the monoclonal antibody(4.6ml, 5.2 mg/ml) were each dialysed against 0.1M potassium phosphate, 0 1 M NaCl, pH 7.5 (coupling buffer) and incubated separately for 2h at room temperature with eightfold molar excess of SPDP (125µl of a 3.2 mg/ml solution of SPDP in ethanol was added to each sample). The monoclonal antibody was redialysed against coupling buffer and the anti-alkaline phosphatase was dialysed against 0.1M sodium acetate, 0.1M Na Cl pH 4.5. Dithiothreitol was then added to the anti-alkaline phosphatase to a final concentration of 0.02M. After 30 minutes at room temperature, the anti-alkaline phosphatase was passed through a Pharmacia PD10 column equilibrated with coupling buffer, and immediately added to the monoclonal antibody. After 4 hr incubation at room temperature 1 mg of iodoacetamide was added and the protein was eluted on a 2.5 x 90cm Ultrogel AcA 22 column in borate buffered saline, pH 8.5 Polymerised material was eluted in two fractions and concentrated using a Millipore CX-100 immersible membrane.

### EXAMPLE 2

### Reduction in Viability of Tumour Cells

A cell line obtained from a human carcinoma was grown and aliquots placed in two microtitre plates at a density of 10,000 cells per well in incorplete modified Dulbecco's medium with 10% foetal calf serum (IMDM). The wells of both plates then received 200µl of a 5µg/ml solution of cAb in IMDM apart from one row which received only IMDM (the zero control'). The plates were then incubated for one hour at 4°C and unbound antibody removed by three change of the culture medium. On plate was then exposed to UV-A light in a similar means to that described in S Thompson et al, Biochem. Biophys. Res. Com., 201,1213-1219 (1994). The pro-drug etoposide phosphate was synthesised as described in Senter, PD., Saulner MG., Schreiber GJ. Hirschberg DL., Brown JP., Heistrom KE, Proc Natl. Acad. Sci. USA, 85, 4842-4846 (1988) from the parent compound etoposide (Sigma Chemical Co. Ltd. 1994 cat no. E 1383) was added over a range of concentrations to groups of wells and incubated at 37°C for 6 hours the cells are then washed a further 12 hours. The cells in the invididual wells are then assessed for their viability and it is seen that the cells in the wells previously illuminated by the UV-A light were not viable That this was an effect due to the binding of alkaline phosphatase in the illuminated wells and consequently a greater activation of the pro-drug in those wells was demonstrated by the cells in the zero control remaining viable after exposure to the UV-A.

### EXAMPLE 3

Example 1 was repeated making the bispecific (F(ab')2 conjugate from F(ab')2 fragments of both the antibodies following the method described in Glennie MJ, Brennand DM, Bryden F, Stirpe F, Woth AT & Stephenson GT (1987), Preparation and performance of bispecific F(ab'γ)2 antibody containing thloether-lined Fab'γ fragments, Journal of Immunology, 139, 2367-2375.

### EXAMPLE 4

The procedure of Example 1 was repeated using the linker M2C2H of the Pierce and Warriner 1995 catalogue (cat. No. 22304) instead of SPDP.

### EXAMPLE 5

The procedure of Example 3 was repeated using the linker M2C2H of the Pierce and Warriner 1995 catalogue (cat. No. 22304) (ie 4-(N-maleimidomethyl)-cyclohexane-1-carboxylhydrazide) instead of SPDP.

### Example 1A

### Association of Alkaline Phosphatase on colon carcinoma cells by means of a bispecific antibody the enzyme binding activity of which was modulatable by ultraviolet light irradiation

### Preparation of the bispecific antibody conjugate

Monoclonal antibodies against Carcinoembrionic antigen (CEA) and alkaline phosphatase (AP) (Zymed Laboratories Inc. clone number ZAP1 cat. no 03-2200) were obtained.

The monoclonal anti-AP was first coated with NPE. The antibody, at a concentration of 0.5mg/ml, was first dialysed overnight against 0.1M NaHCO3. During dialysis the volume increased from 3 to 4ml. 1ml was retained as a control and two 1.5ml fractions taken. Nitrobenzylcarbonyl chloride was prepared by treating NPE with di-phosgene in dioxan [Senter, PD, Tansey, MJ, Lambert, JM & Blattler, WA (1985), Photochem. Photobiol., 42, 231-237] and 10µl of this was added to one 1.5ml fraction and 20µl was added to the other. The samples were allowed to react with gentle rotation for 4hr and were then dialysed against 0.9M NaCl. The final concentration of the control sample was 0.21mg/ml and of the treated samples 0.135 and 0.105mg/ml. The samples were found to have been coupled to around the same extent (averaging 8 and 12 NPE residues per antibody molecule) and were consequently mixed. The coated antibody was used without further purification. Both the NPE-coated anti-AP antibody and the anti-CEA antibody at 0.18ml/ml were derivatised with SPDP: 1ml aliquots of both antibodies were dialysed at a concentration of 0.1ml/ml against 0.1M phosphate buffer pH 7.5 containing 0.1M NaCl. 25µl of SPDP (0.63ml/ml in ethanol) was then added to each antibody. The antibodies were then left for 30 minutes to react. The anti-AP was dialysed against 0.1M phosphate buffer pH 7.5 and the anti-CEA antibody and left for 30 minutes at room temperature. The preparation was dialysed against two batches of 0.1M phosphate buffer pH 7.5. The anti-CEA antibody was then reduced and added to the NPE-anti-AP antibody and left to react for 24 hours at 20°C The conjugate was then dialysed against distilled water to remove unwanted reaction products. The yield was shown by electrophoresis to be ca. 80% with at least 75% of the antibodies conjugated.

### Targeting of AP to CEA-coated ELISA plates

Nunc microtitre plates were coated with 10µg/ml CEA (Calbiochem 1994/5 cat. no 219368). The bispecific antibody conjugate was then either irradiated or not for 6 minutes with UV light and 50µl of each at 10µg/ml were diluted to 1ml in Tris buffer pH 7.4 containing 0.05% Tween and doubling dilutions added to wells across the plate. Both native anti-CEA and anti-AP antibodies were similarly treated as controls. After two hours the plate was washed and 100µl AP at 2µg/ml or, in control wells, an anti-mouse lgG labelled with alkaline phosphatase (at 1:1000 dilution) was added in Tris-Tween buffer. The AP (Biogenesis cat. no. 0300-1004) was added to directly detect the presence of active conjugate which had bound to the CEA, the anti-IgG(AP) as a positive control to confirm the SPDP coated anti-CEA antibody (either conjugated or not) could still bind to the CEA on the plate. The AP was visualised in all wells by the addition of nitro-phenolphosphate substrate under standard conditions and the reaction monitored spectrophotometrically at 405nm. This showed that irradiating the bispecific antibody resulted in more AP being bound the microtitre plate wells than was bound with non-irradiated bispecific antibody.

### Targeting of AP to colon cancer cells expressing CEA

CEA producing colon cancer cells were grown in microtitre plates to approximately 90% confluence, this time in 200µl DMEM medium per well containing 10% foetal calf serum. 100µl of the medium was then removed and replaced with free serum free medium for 1hr to accustom the cells to serum free media (SFM). All media was then removed and 100µl of SFM containing the bispecific antibody conjugates at 2.5µg/ml which had either not been irradiated or after irradiation by 2ml of the bispecific antibody conjugate sample being irradiated in saline for 6 minites in a quartz cuvette at a distance of 0.5cm from a spectroline EN/16/F UV lamp (Spectronics Corporation, Westbury, New York) with an emission peak of 365 nm. The assay was performed with 10 replicate wells in each case After 2hrs all of the media was again removed and 100µl of SFM containing AP (2µ g/ml) was added. After a final 2hr incubation the cells were extensively washed (five times) with buffered saline and 100µl of substrate buffer containing AP substrate pNPP was added and the AP activity then monitored at 405nm with a microtitre plate reader Controls were added in which no bispecific conjugate was added and also in which bispecific conjugate had been added but no AP This provided a measurement of the natural levels of AP in the cells and also allowed us to prove that the cells to which AP had been added had been washed sufficiently to remove all non-specifically absorbed AP.

The results showed that the wells containing unirradiated bispecific conjugate only showed a background level of AP which was the same as wells not receiving the bispecific antibody or added AP. In contrast, the wells which had received the irradiated bispecific antibody conjugate demonstrated much higher levels of AP activity as a result of this being bound to the CEA producing cells throught the active bispecific antibody conjugate.

### Example 2A

### Irradiation in culture medium

Example 1A (last section) was repeated with an enzyme amplified system employing an AMPAK kit according to the manufacturer's instructions for the detection of AP in microtitre plate assays (Dako Diagnostics Ltd. U.K.) which produced a readily identifiable red colour in place of the para-nitrophenolphosphate to investigating any possible effect of culture medium during the irradiation of bispecific antibody conjugate. In this the conjugate, at 40µg/ml was irradiated in quartz cuvettes in 0.9% saline or SFM for 6 minutes. The antibodies were then diluted to 5µg/ml in either SFM or media contianing 10% FCS before being added to 10-fold replicate wells contianing the CEA-producing colon cancer cells. Table 1 shows the visually scored results of this from which it was clear that much more AP was bound by the wells receiving irradiated bispecific antibody conjugate, irrespective of whether the irradiation had been carried out in SFM or saline, than those which had received the unirradiated bispecific antibody conjugate.

**Table 1 The visual results were scored from 0 to a maximum of 10**

| | |
|---|---|
| Plate Blank | |
| No irradiation | 3 |
| Irradiation in saline | 10 |
| lrradiation in SFM | 10 |

### Example 3A

### Time of irradiation

Example 1A (last section) was repeated but here, the enzyme amplified system was used as in Example 2A and also the unirradiated bispecific antibody conjugate at concentrations of 2.5µg/ml and 5.0µg/ml was added directly to all of the wells of the plate after which the cells were irradiated from below the plate for times of: 0, 3, 6, 9 and 12 minutes. As shown by the results in Table 2, even with the attenuation of the irradiation by the plastic plate, increasing AP capture was strongly related to increasing time of irradiation.

**Table 2 The results were scored from 0 to a maximum of 10 as follows:**

| Irradiation Time (min) | conjugate Visual Colour 2.5µg/ml development | conjugate Visual Colour 5.0µg/ml development |
|---|---|---|
| 0 | 1 | 4 |
| 3 3 | 3 | 5 |
| 6 | 7 | 8 |
| 9 | 8 | 10 |
| 12 | 10 | 10 |

### Example 3B

Phosphorylated etoposide is obtained as described by Haisma et al in Cancer Immunol. Immunother. (1992) 34:343-348. Example 2A is repeated but instead of the enzyme amplified system being addded etoposide phosphate is added as described in Haisma et al. Tumour cell death is greater in those cultures not exposed to the irradiated bispecific conjugate than to the unirradiated bispecific conjugate.

The CEA producing cells are grown in flasks and accustomed to serum-free medium as in Example 1A. They are then plated in microtitre plates and a solution of 100µg/ml in serum-free culture fluid added to half the wells. The other half receives the same bispecific antibody preparation but which has been irradiated through 2ml of the NPE-coated bispecific antibody sample being irradiated in serum-free culture fluid for 6 minutes in a quartz cuvette at a distance of 0.5cm from the Spectroline lamp. The cells are incubated for 1hr at 37°C after which they are washed by gentle centrifugation and change of medium. 100µ SFM containing alkaline phosphatase at 2µg/ml is added to each well and inculated for 30 minutes at 37°C after which they are washed by gentle centrifugation and a solution of 100µl epoposide phosphate (10µM), made according to Senter et al Proc. Natl. Acad. Sci. USA (1988) 85: 4842, in SFM added to each well and incubated at 37°C for 3hr. The cells are again washed and grown for three days whereupon their viability is assessed by the sulphorhodamine-B assay. It is found that those wells having received irradiated bispecific antibody show lower cell viability than those receiving unirradiated antibody.

### In-situ Irradiation

The CEA producing cells are grown in flasks and accustomed to serum-free medium as in Example 1A. They are then plated in microtitre plates and a solution of 100µl NPE-coated bispecific antibody made as in Example 1 at a concentration of 10µg/ml in serum-free culture fluid is added to the wells Half of the wells are irradiated as described in Example 3A. The cells are incubated for 1hr at 37°C after which they are washed by gentle centrifugation and change of medium 100µl SFM containing alkaline phosphatase at 2µg/ml is added to each well and incubated for 30 minutes at 37°C after which they are washed again and a solution of 100µl epoposide phosphate (10µM), made according to Senter et al Proc. Natl. Acad. Sci. USA (1988) 85: 4842, in SFM added to each well and incubated at 37°C for 3hr. The cells are again washed and grown for three days whereupon their viability is assessed. Control wells are also set up which do not receive either the NPE-bispecific antibody, alkaline phosphatase or eptoposide phosphate or combinations of the three. When the cell viability is assessed by the sulphorhodamine-B assay as described by Skehan et al (1990) New colorimetric cytotoxicity assay for anticancer-drug screening, J. Natl. Cancer Inst. 82: 1107 those wells having been given the NPE-bispecific antibody, alkaline phosphatase and etoposide phosphate and then exposed to the UV light show greater cell death than those in unirradiated wells or in control wells irradiated or not.

### Example 4A

### Use of a bispecific antibody having a reversibly inhibited binding site for alkaline phosphatase and binding affinity for carcinoembrionic antigen to locate alkaline-phosphatase-bound particulate beads with cells bearing carcinoembrionic antigen after the bispecific antibody had been uninhibited by irradiation with ultraviolet light

### Preparation of the bispecific antibody conjugate

Monoclonal antibodies against Carcinoembrionic antigen (CEA) and alkaline phosphatase (AP)(Zymed Laboratories Inc. clone number ZAP1 cat. no. 03-2200) were obtained.

The monoclonal anti-AP was first coated with NPE The antibody, at a concentration of 0.5mg/ml, was first dialysed overnight against 0.1M NaHNO3. During dialysis the volume increased from 3 to 4ml. 1ml was retained as a control and two 1.5ml fractions taken. Nitrobenzylcarbonyl chloride was prepared by treating NPE with di-phosgene in dioxan [Senter, PD, Tansey, MJ, Lambert, JM & Blattler, WA (1985), Photochem Photobiol., 42. 231-237] and 10µl of this was added to one 1.5ml fraction and 20µl was added to the other The samples were allowed to react with gentle rotation for 4hr and were then dialysed against 0.9M NaCl. The final concentration of the control sample was 0.21 mg/ml and of the treated samples 0.135 and 0.105mg/ml. The samples were found to have been coupled to around the same extent (averaging 8 and 12 NPE residues per antibodymolecule) and were consequently mixed. The coated antibody was used without further purification. Both the NPE-coated anti-AP antibody and the anti-CEA antibody at 0. 18mg/ml were derivatised with SPDP: 1 ml aliquots of both antibodies were dialysed at a concentration of 0. 1mg/ml against 0.1M phosphate buffer pH 7.5 containing 0.1M NaCl. 24µl of SPDP (0.63mg/ml in ethanol) was then added to each antibody. The antibodies were then left for 30 minutes to react. The anti-AP was dialysed against 0.1M phosphate buffer pH 7.5 and the anti-CEA was dialysed against 0.1M sodium acetate pH 4.5 both being dialysed overnight at 4°C. After dialsysis 100µl of 0.5M DL-dithioihreitol (DTT) was added to the anti-CEA antibody and left for 30 minutes at room temperature. The preparation was dialysed against two batches of 0.1M phosphate buffer pH 7.5. The anti-CEA antibody was then reduced and added to the NPE-anti-AP antibody and left to react for 24 hours at 20°C. The conjugate was then dialysed against distilled water to remove unwanted reaction products. The yield was shown by electrophoresis to be ca. 85% with at least 75% of the antibodies conjugated.

### Preparation of the beads

Carboxylate modified intensely blue coloured polystyrene beads of three different sizes (0.22 mm, 0.4 mm and 0.8 mm) were obtained. The beads were coupled with alkaline phosphatase (AP) by first washing them three times by resuspending 100ml of 10% beads in 1 ml of distilled water and pelleted by centrifugation at 10000 RPM for 10 minutes. The beads were then resuspended in 120ml of MES buffer in 770ml distilled water with 230ml of N-hydroxysuccinime at 50mg/ml in distilled water (NHS), 1-Ethyl-3-(3-dimethylaminopropyl) carbodiimide hydrochloride (EDAC) at 50mM/ml was prepared and 100µl immediately added to the bead preparation the beads were then left for 2 hours and washed once with 0.1 MES buffer pH 4 7 and then with 0.1M bicarbonate pH 8.4. 500µl of AP at 2mg/ml was then added to the pelleted beads. The volume was adjusted to 1 ml in the same buffer and left to react overnight. The next day the beads were washed three times, centrifuged in microcenttifugation-tubes and then resuspended in 1ml phosphate buffered saline.

The protein concentration was calculated for each set of beads. 80% of added AP was found to have bound to each set.

### Capture of AP-labelled beads by activated bispecific antibody conjugate on cells

CEA producing colon cancer cells were grown in microtitre plates to approximately 90% confluence, this time in 200µ DMEM medium per well containing 10% foetal calf serum 100µ of the medium was then removed and replaced with free serum free medium for 1hr to accustom the cells to serum free media (SFM). All media was then removed and 100µl of SFM containing the bispecific antibody conjugates at 2.5µg/ml which had either not been irradiated or after irradiation by 2ml of the bispecific antibody conjugate sample being irradiated in saline for 6 minutes in a quartz cuvette at a distance of 0.5cm from a spectroline EN/16/F UV lamp (Spectronics Corporation, Westbury, New York) with an emission peak of 365nm. The assay was performed with 10 replicate wells in each case. After 2hr all of the media was again removed and 10µl of each bead suspension followed by 90µl of serum free medium added to their wells and left for 24hr. The plate was emptied, washed 5 times with phosphate buffered saline and 100µl of 5mM para-nitrophenol phosphate in 50mM bicarbonate buffer pH 10.3 containing 3.3mM MgCl₂ buffer was added and the enzyme product measured at 405nm with a microtitre plate reader This was confirmed by measurement of the alkaline phosphatase activity on the beads where with all three bead sizes more activity was found associated with those wells which had received irradiated conjugate than those which had not.

### Example 5A

An anti-CD3 (T lymphocyte activating marker) monoclonal antibody is coated with NPE and coupled to the anti-CEA monoclonal antibody was was the anti-alkaline phosphatase antibody in Example 1A. This is then divided into two aliquots, one being irradiated as in Example 1A and the other untreated. They are then compared in their ability to target T-cells obtained and cause lysis of the human cancer cells as described in Barr et al, Int. J. Cancer (1989), 43, 501-507 but employing the concentrations of antibody conjugate described in Example 1A above (Targeting of AP to colon cancer cells expressing CEA). More cell death is obtained with the irradiated fraction of the bispecific antibody than that not irradiated.

### In Situ Targeting

The NPE-coated anti-CD3 - anti-CEA bispecific antibody conjugate is employed in situ activation as follows: They are then compared in their ability to target T-cells obtained and cause lysis of the human cencer cells as described in Barr et al. The CEA-bearing cells are plated and accustomed to serum free medium as described in Example 1A above (Targeting of AP to colon cancer cells expressing CEA). Antibody conjugate is added in serum-free medium (SFM) at a concentration of 2.5µg/ml and incubated at 37°C for two hours, other wells being control wells and not recieving antibody conjugate. Wells are irradiated as described in Example 3A and the cells are washed in SFM. The ability of added T-cells to lyse the CEA-expressing cells is then assessed following Barr et al. More cell lysis is obtained in irradiated wells than those not irradiated or those control wells not receiving the bispecific antibody, whether or not irradiated.

### Lists of Receptors and their Ligands

The receptor may be
neurotransmitter receptors
gastric receptors such as enterogastrone, cholecystokinin, pancreozymin and secretin histamine receptors, serotonin receptors
alpha and beta-adrenoceptors
aldrenalin receptors, noradrenalin receptors
enkephalis receptors, endorphin receptors
melanine stimulating hormone receptor
acetylcholine receptor
other homone receptors such as insulin receptors and growth hormone receptors
thyroxin, thyrotropin, glucagon, progesterone, oestradiol, testosterone, folcile stimulating hormone, luteinizing hormone, chorionic gonadotropin, placental lactogen, oxytocin, vasopressin, erythropoietin, renin and angiotenin, ACTH receptors
corticosterone, cortisol, 5α-dihydrotestosterone, aldosterone, 1.25-dihydroxycholecalciferol, parathyroid hormone and calcinonin receptors
corticorophin releasing factor receptors
cytokine receptors such as Tumour Necrosis Factor Receptor
Immune receptors such as Histocompatability antigen receptors, T-cell receptors and B-cell receptors
specific drug receptors such as diethylstilbestrol (oesradiol-17β receptor)
specific carbohydrate structures
enzymes may be used as receptors
synthetic receptors are being increasingly made

The ligand may be
gastric hormones and factors such as:
enterogastrone, cholecystokinin, parcreozymin, histamin and secretin neurotransmitters such as: serotonin, adrenalin, noradrenalin, enkephalins, endorphins, melanine stimulating hormone, acetylcholine, insulin receptors and growth hormone, thyroxin, thyrotropin, glucagon, progesterone, oestradiol, testosterone, folcile stimulating hormone, luteinizing hormone, chorionic gonadotrophin, placental lactogen, oxytocin, vasopressin, erythropoietin, renin and angiotensin, ACTH, corticosterone, cortisol, 5α-dihvdrotestosterone, aldosterone, 1,25-dihydroxycholecalciferol, parathyroid hormone and calcitonin, corticorophin releasing factor, cytokines such as Tumour Necrosis Factor, Immune agents such as Histocompabability antigens, T-cell factors and B-cell factors and antigens, specific drugs such as diethylstilbestrol (oesradiol-17β receptor)
enzyme substrates, especially irreversible inhibitors
ligands for synthetic receptors
specific carbohydrate binding agents such as: Wheat-germ agglutin, Pokeweed mitrogen (may be ligands or receptor)

## Claims

1. An antibody for use in a method of medical treatment, wherein
(a) the antibody is labelled with a member of a binding pair consisting of a ligand and a receptor, so that the antibody is capable of binding to the other member of the binding pair when present on the surface of a target cell which it is desirable to eliminate, and
(b) the antibody is capable of binding to an enzyme which is capable of converting a pro-drug of a cytotoxic drug into the cytotoxic drug, or to a cell of the immune system which can kill said target cell;
wherein the binding of the antibody to the enzyme or cell of the immune system is reversibly inhibited by the presence of a photocleavable moiety.

2. A bispecific antibody for use in a method of medical treatment, the bispecific antibody comprising a first antibody component which is capable of binding to a member of a binding pair consisting of a ligand and a receptor present on the surface of a target cell which it is desirable to eliminate, and a second antibody component which is capable of binding to an enzyme which is capable of converting a pro-drug of a cytotoxic drug into the cytotoxic drug, or to a cell of the immune system which can kill said target cell;
wherein the binding of the antibody to the enzyme or cell of the immune system is reversibly inhibited by the presence of a photocleavable moiety.

3. The bispecific antibody of claim 2, wherein the first and second antibody components are parts of antibodies which retain the active site but are free of the Fc regions.

4. The antibody of any one of the preceding claims, wherein the photocleavable moiety is nitrophenyl methyl alcohol, 1-nitrophenylethan-1-ol or 1-(2-nitrophenyl)ethanol conjugated to the antibody.

5. The antibody of any one of the preceding claims, wherein the target cell is a tumour cell.

6. The antibody of any one of the preceding claims, wherein the enzyme is a phosphatase, a carboxypeptidase, a β-glucosidase, a β-lactamase, an amidase, a cytosine deaminase or a nitroreductase.

7. The antibody of any one of the preceding claims, wherein the cell of the immune system is a natural killer cell, a T-cell, a cytotoxic T-cell, a macrophage, an activated peripheral blood lymphocyte, a CD3+ lymphocyte, a CD16+ lymphocyte, a polymorphonuclear leucocyte, a large granular lymphocyte, a B lymphocyte, a myeloid cell, a lymphokine activated killer cell or a monocyte.

8. Use of an antibody of any one of the preceding claims for the preparation of a medicament for the treatment of a tumour.

9. The use of claim 8, wherein electromagnetic energy is used to remove the photocleavable moiety and activate the ability of the antibody to bind the enzyme or cell of the immune system.

10. An antibody for use in a method of medical treatment, wherein the antibody is a bispecific antibody capable of binding to
(a) a target cell which it is desirable to eliminate; and
(b) to a virus;
wherein the binding of the antibody to the virus is reversibly inhibited by the presence of a photocleavable moiety.

11. The antibody of claim 10, wherein the target cell is a tumour cell.

12. A kit for use in a method of medical treatment, the kit comprising:
(i) a cell which is a carrier of a cytotoxic agent, a radioactive isotope, a toxin, a virus or boron; and
(ii) an antibody capable of binding to
(a) a member of a binding pair consisting of a ligand or a receptor present on the surface of a target cell which it is desirable to eliminate; and
(b) said cell which is a carrier of a cytotoxic agent, a radioactive isotope, a toxin, a virus or boron;
wherein the binding of the antibody to the carrier cell is reversibly inhibited by the presence of a photocleavable moiety.

## Patentansprüche

1. Antikörper zur Verwendung in einem medizinischen Behandlungsverfahren, worin
(a) der Antikörper mit einem Element eines Bindungspaars markiert ist, das aus einem Liganden und einem Rezeptor besteht, sodass der Antikörper in der Lage ist, sich an das andere Element des Bindungspaares zu binden, wenn an der Oberfläche einer Targetzelle vorhanden, die wünschenswerterweise zu entfernen ist, und
(b) der Antikörper in der Lage ist, sich an ein Enzym, das in der Lage ist, ein Prodrug eines zytotoxischen Wirkstoffs zum zytotoxischen Wirkstoff umzusetzen, oder an eine Zelle des Immunsystems, die die Targetzelle töten kann, zu binden;
worin die Bindung des Antikörpers an das Enzym oder die Zelle des Immunsystems durch die Gegenwart einer photospaltbaren Gruppierung reversibel inhibiert ist.

2. Bispezifischer Antikörper zur Verwendung in einem medizinischen Behandlungsverfahren, worin der bispezifische Antikörper eine erste Antikörperkomponente, die in der Lage ist, sich an ein Element eines Bindungspaars zu binden, das aus einem Liganden und einem Rezeptor besteht, vorhanden an der Oberfläche einer Targetzelle, die wünschenswerterweise zu entfernen ist, und eine zweite Antikörperkomponente umfasst, die in der Lage ist, sich an ein Enzym, das in der Lage ist, ein Prodrug eines zytotoxischen Wirkstoffs zum zytotoxischen. Wirkstoff umzusetzen, oder an eine Zelle des Immunsystems, die die Targetzelle töten kann, zu binden;
worin die Bindung des Antikörpers an das Enzym oder die Zelle des Immunsystems durch die Gegenwart einer photospaltbaren Gruppierung reversibel inhibiert ist.

3. Bispezifischer Antikörper nach Anspruch 2, worin die erste und die zweite Antikörperkomponente Teile von Antikörpern sind, die die aktive Stelle beibehalten, jedoch frei von Fc-Regionen sind.

4. Antikörper nach einem der vorangehenden Ansprüche, worin die photospaltbare Gruppierung Nitrophenylmethylalkohol, 1-Nitrophenylethan1-ol oder 1-(2-Nitrophenyl)ethanol, konjugiert an den Antikörper, ist.

5. Antikörper nach einem der vorangehenden Ansprüche, worin die Targetzelle eine Tumorzelle ist.

6. Antikörper nach einem der vorangehenden Ansprüche, worin das Enzym eine Phosphatase, eine Carboxypeptidase, eine β-Glucosidase, eine β-Lactamase, eine Amidase, eine Cytosindesaminase oder eine Nitroreductase ist.

7. Antikörper nach einem der vorangehenden Ansprüche, worin die Zelle des lmmunsystems eine natürliche Killerzelle, eine T-Zelle, eine zytotoxische T-Zelle, ein Makrophage, ein aktivierter peripherer Blutlymphozyt, ein CD3+-Lymphozyt, ein CD16+-Lymphozyt, ein polymorphkerniger Leukozyt, ein großer granulärer Lymphozyt, ein B-Lymphozyt, eine Knochenmarkzelle, eine Lymphokin-aktivierte Killerzelle oder ein Monozyt ist.

8. Verwendung eines Antikörpers nach einem der vorangehenden Ansprüche zur Herstellung einer Medikaments zur Behandlung eines Tumors.

9. Verwendung nach Anspruch 8, worin elektromagnetische Energie verwendet wird, um die photospaltbare Gruppierung zu entfernen und die Fähigkeit des Antikörpers zu aktivieren, das Enzym oder die Zelle des Immunsystems zu binden.

10. Antikörper zur Verwendung in einem medizinischen Behandlungsverfahren, worin der Antikörper ein bispezifischer Antikörper ist, der in der Lage ist,
(a) eine Targetzelle, die wünschenswerterweise zu entfernen ist; und
(b) ein Virus zu binden;
worin die Bindung des Antikörpers an das Virus durch die Gegenwart einer photospaltbaren Gruppierung reversibel inhibiert ist.

11. Antikörper nach Anspruch 10, worin die Targetzelle eine Tumorzelle ist.

12. Set zur Verwendung in einem medizinischen Behandlungsverfahren, umfassend:
(i) eine Zelle, die ein Träger eines zytotoxischen Mittels, eines radioaktiven isotops, eines Toxins, eines Virus oder von Bor ist; und
(ii) einen Antikörper, der in der Lage ist, sich
(a) an ein Element eines Bindungspaars zu binden, das aus einem Liganden oder einem Rezeptor besteht, an der Oberfläche einer Targetzelle vorhanden, die wünschenswerterweise zu entfernen ist; und
(b) an die Zelle zu binden, die ein Träger eines zytotoxischen Mittels, eines radioaktiven Isotops, eines Toxins, eines Virus oder von Bor ist;
worin die Bindung des Antikörpers an die Trägerzelle durch die Gegenwart einer photospaltbaren Gruppierung reversibel inhibiert ist.

## Revendications

1. Anticorps à utiliser dans une méthode de traitement médical, où
(a) l'anticorps est marqué avec un membre d'une paire de liaison consistant en un ligand et un récepteur, de façon que l'anticorps soit capable de liaison à l'autre membre de la paire de liaison quand il est présent à la surface d'une cellule cible qu'il est souhaitable d'éliminer, et
(b) l'anticorps est capable de liaison à une enzyme qui est capable de convertir un promédicament d'un médicament cytotoxique en le médicament cytotoxique, ou à une cellule du système immun qui peut tuer ladite cellule cible;
où la liaison de l'anticorps à l'enzyme ou à la cellule du système immun est inhibée réversiblement par la présence d'une fraction photoscindable.

2. Anticorps bispécifique à utiliser dans une méthode de traitement médical, l'anticorps bispécifique comprenant un premier composant d'anticorps qui est capable de liaison à un membre d'une paire de liaison consistant en un ligand et un récepteur présent à la surface d'une cellule cible qu'il est souhaitable d'éliminer et un second composant d'anticorps qui est capable de liaison à une enzyme qui est capable de conversion en un promédicament d'un médicament cytotoxique en le médicament cytotoxique, ou à une cellule du système immun qui peut tuer ladite cellule cible;
où la liaison de l'anticorps à l'enzyme ou à la cellule du système immun est réversiblement inhibée par la présence d'une fraction photoscindable.

3. Anticorps bispécifique de la revendication 2, où les premier et second composants d'anticorps sont des parties d'anticorps qui conservent le site actif mais sont sans les régions Fc.

4. Anticorps de l'une quelconque des revendications précédentes, où la fraction photoscindable est l'alcool nitrophényl méthylique, le 1-nitrophényléthan-1-ol ou le 1-(2-nitrophényl)éthanol conjugué à l'anticorps.

5. Anticorps de l'une quelconque des revendications précédentes, où la cellule cible est une cellule de tumeur.

6. Anticorps de l'une quelconque des revendications précédentes, où l'enzyme est une phosphatase, une carboxypeptidase, une β-glucosidase, une β-lactamase, une amidase, une cytosine désaminase ou une nitroréductase.

7. Anticorps de l'une quelconque des revendications précédentes, où la cellule du système immun est une cellule tueuse naturelle, une cellule T, une cellule T cytotoxique, un macrophage, un lymphocyte de sang périphérique activé, un lymphocyte CD3+, un lymphocyte CD16+, un leucocyte polymorphonucléaire, un grand leucocyte granulaire, un lymphocyte B, une cellule myéloïde, une cellule tueuse activée par une lymphokine ou un monocyte.

8. Utilisation d'un anticorps de l'une quelconque des revendications précédentes pour la préparation d'un médicament pour le traitement d'une tumeur.

9. Utilisation de la revendication 8, où une énergie électromagnétique est utilisée pour éliminer la fraction photoscindable et activer l'aptitude de l'anticorps à lier l'enzyme ou la cellule du système immun.

10. Anticorps à utiliser dans une méthode de traitement médical, où l'anticorps est un anticorps bispécifique capable de liaison à
(a) une cellule cible qu'il est souhaitable d'éliminer; et
(b) à un virus;
où la liaison de l'anticorps au virus est réversiblement inhibée par la présence d'une fraction photoscindable.

11. Anticorps de la revendication 10, où la cellule cible est une cellule tumeur.

12. Kit à utiliser dans une méthode de traitement médical, le kit comprenant:
(i) une cellule qui est une porteuse d'un agent cytotoxique, un isotope radioactif, une toxine, un virus ou du bore; et
(ii) un anticorps capable de liaison à
(a) un membre d'une paire de liaison consistant en un ligand ou un récepteur présent à la surface d'une cellule cible qu'il est souhaitable d'éliminer; et
(b) ladite cellule qui est un support d'un agent cytotoxique, un isotope radioactif, une toxine, un virus ou du bore;
où la liaison de l'anticorps à la cellule porteuse est réversiblement inhibée par la présence d'une fraction photoscindable.
